**Europäisches Patentamt**

(19) **European Patent Office**

**Office européen des brevets**

(11) Numéro de publication: **0 108 667**

**A1**

# (12) DEMANDE DE BREVET EUROPEEN

(21) Numéro de dépôt: 83401969.7

(22) Date de dépôt: 07.10.83

(51) Int. Cl.³: **C 12 N 15/00**
C 12 P 21/02, A 61 K 37/36
C 07 G 7/00

(30) Priorité: 07.10.82 FR 8216819

(43) Date de publication de la demande:
16.05.84 Bulletin 84/20

(84) Etats contractants désignés:
AT BE CH DE FR GB IT LI LU NL SE

(71) Demandeur: INSTITUT PASTEUR (FONDATION)
28, rue du Docteur Roux
F-75715 Paris Cedex 15(FR)

(72) Inventeur: Roskam, Willem Gerrit
7, Allée de la Forêt de Marly
F-78860 Saint nom la Breteche(FR)

(72) Inventeur: Lupker, Johannes Hermanus
41, rue de Marnes
F-92410 Ville d'Avray(FR)

(72) Inventeur: Miloux, Brigitte Renée
4, rue Parmentier Lozère
F-91120 Palaiseau(FR)

(72) Inventeur: Yaniv, Moshé
159, rue Blomet
F-75015 Paris(FR)

(72) Inventeur: Liauzun, Philippe Pierre
135, rue Didot
F-75014 Paris(FR)

(72) Inventeur: Jouanneau-Gailliard, Jacqueline
21, rue Charcot
F-75013 Paris(FR)

(74) Mandataire: Gutmann, Ernest et al,
Cabinet Plasseraud 84, rue d'Amsterdam
F-75009 Paris(FR)

(54) **Lignées cellulaires originaires de mammifères, productrices d'hormone de croissance, de préférence humaine, et vecteurs pour leur obtention.**

(57) L'invention concerne des lignées de cellules de mammifères, notamment de singe, qui sont capables de produire une hormone de croissance, de préférence humaine et de l'excréter dans leur milieu de culture.

Ces lignées sont modifiées par un fragment d'ADN contenant une séquence codant pour cette hormone de croissance et, de préférence aussi, une séquence codant pour un peptide signal associé à cette hormone de croissance, immédiatement en amont de la précédente vis-à-vis de la direction de la transcription, l'expression de ce fragment étant sous le contrôle d'un promoteur exogène également incorporé de façon stable dans lesdites cellules.

EP 0 108 667 A1

1

Lignées cellulaires originaires de mammifères, productrices d'hormone de croissance, de préférence humaine, et vecteurs pour leur obtention.

———————

L'invention concerne de nouvelles lignées stables de cellules d'eucaryotes supérieurs, plus particulièrement de mammifère, capables de synthétiser et d'excréter une hormone qui possède les propriétés essentielles de l'hormone de croissance.

Elle concerne aussi des vecteurs contenant une séquence codant pour une protéine ayant les propriétés d'une hormone de croissance de mammifère, notamment d'une hormone de croissance humaine (hGH), cette protéine étant susceptible d'être exprimée dans des cellules d'eucaryotes supérieurs, plus particulièrement de mammifères, et antérieurement inaptes à synthétiser une telle protéine.

L'hormone de croissance (hGH), synthétisée par l'hypophyse, est nécessaire à la croissance de l'enfant. Seule l'hormone humaine est active chez l'homme et elle est actuellement utilisée dans le traitement du nanisme hypophysaire. L'hormone est obtenue par extraction à partir d'hypophyses de cadavres. Cette source limitée ne permet pas de satisfaire les besoins thérapeutiques, ni de confirmer et d'exploiter d'autres indications telles que le traitement de brûlures, de fractures, et des ulcères d'estomac. La structure de l'hormone principale est connue depuis longtemps (Li, C. H., Dixon, J.S. and Chung, D. (1973) Arch. Biochem. Biophys. 155, 95-110), mais il est possible que des hormones voisines existent (Lewis, U.J., et al. (1978), J. Biol. Chem. 253,

0108667

2

2679-2687).

L'hormone de croissance humaine est synthétisée dans des cellules de l'hypophyse antérieure comme préhormone (Roskam, W.G. et Rougeon, F., 1979, Nucl. Acids Res. 7, 305-320) comportant à l'extrémité $NH_2$ une extension de 26 acides aminés. Cette extension est enlevée lors de l'excrétion pour donner l'hormone active circulante de 191 acides aminés.

Les techniques du génie génétique ont été appliquées au clonage du gène de l'hormone de croissance humaine (clonage du cDNA : Roskam, W.G. et Rougeon, F. op. cit. ; Goeddel, D.V. et al. (1979) Nature 201, 544-548; Martial, J.A. et al. (1979) Proc. Natl. Acad. Sci. USA 76, 4294-4298). Le cDNA cloné a été manipulé pour en obtenir l'expression dans E. coli. L'équipe de Goodman (Martial, JA. et al. (1979) op. cit.) décrit la synthèse d'une protéine hybride contenant une séquence bactérienne. L'équipe de Genentech (Goeddel, D.V. et al. (1979) op. cit.) décrit la synthèse de méthionine-hGH, contenant à l'extrémité $NH_2$ terminale une méthionine supplémentaire par rapport à la protéine naturelle. L'aminoacide supplémentaire est la conséquence de l'insertion d'un codon d'initiation (ATG) au début du gène codant pour l'hormone naturelle. Il est évident que seule une expérimentation en clinique à long terme pourra confirmer que la méthionine-hGH n'a pas des propriétés indésirables par rapport à l'hormone naturelle et éventuellement infirmer les craintes que certains auteurs ont exprimées. Au surplus la purification de méthionine-hGH à partir d'extraits de E. coli s'est avérée nettement plus difficile que celle de hGH à partir d'hypophyses, notamment en ce qui concerne l'élimination de contaminants bactériens, sources d'effets secondaires indésirables.

Il a certes déjà été proposé de modifier des cellules animales et de les rendre aptes à produire de

3

l'hGH. Ainsi Pavlakis et ses collaborateurs (1981, Proc. Natl. Acad. Sci. USA, 78, 7398) ont réalisé l'introduction du gène chromosomal de hGH, placé sous le contrôle d'un promoteur de SV40 et la production de la protéine, à l'occasion de la réalisation d'un cycle lytique, lequel entraîne alors la lyse de la cellule et la production de particules virales. Un tel mode de production de la protéine serait naturellement inacceptable sur le plan pharmaceutique.

On a également décrit la cotransformation de fibroblastes de souris, initialement déficients en ce qu'ils ne sont pas capables de synthétiser la thymidine-kinase (cellules tk⁻), d'une part, avec un recombinant contenant le gène chromosomal codant pour l'hormone de croissance y compris son promoteur et, d'autre part, avec un marqueur de sélection contenant un gène de la thymidine-kinase (tk). Les cellules transformées se sont révélées capables de synthétiser des quantités détectables d'une protéine ayant les propriétés de l'hormone de croissance, les quantités produites pouvant être accrues en présence d'inducteurs constitués par des glucocorticoïdes (D.M. Robins, et al. (1982), Cell 29, 623-631).

L'introduction du gène chromosomal de l'hormone de croissance de rat dans des fibroblastes de souris, par l'intrermédiaire d'un vecteur contenant l'ADN d'un rétrovirus a également été décrite. L'ADN du rétrovirus confère un phénotype transformé aux cellules ayant intégré ce phénotype transformé, permettant ainsi la sélection de clones ayant intégré le gène de l'hormone de croissance. Mais l'expression du gène de l'hormone de croissance était réalisée sous le contrôle de son propre promoteur et impliquait une régulation de glucocorticoïdes (Doehemer, J. et al. (1982) Proc. Natl. Acad. Sci. USA, 2268-2272).

Les quantités produites, même en présence de

4

tels inducteurs, sont cependant trop faibles pour que cette technique puisse être envisagée au niveau industriel. Au surplus, les cellules utilisées sont tumorigènes, de sorte que leur utilisation pour la production de substances à caractère thérapeutique est a priori exclue.

L'invention a donc pour but de remédier aux inconvénients s'attachant à toutes les techniques antérieures. Plus particulièrement, elle s'est fixée les objectifs que constituent les obtentions de lignées stables de cellules eucaryotes supérieures, notamment de mammifères, capables de produire des quantités importantes d'une protéine ayant l'essentiel de la structure et les propriétés d'une hormone de croissance de mammifère (GH), notamment d'une GH humaine (hGH), ces lignées devant en outre être cultivables industriellement et susceptibles de correspondre aux exigences des autorités responsables de la santé publique.

Les lignées selon l'invention consistent en lignées stables de cellules de mammifères, notamment de singe, dans le génome desquelles est intégré de façon stable, ou dans lesquelles s'exprime de façon stable, un fragment d'ADN contenant une première séquence codant pour une protéine ayant les propriétés d'une hormone de croissance et, de préférence aussi, une seconde séquence codant pour un peptide signal, tel que celui qui est normalement associé à l'hormone de croissance, immédiatement en amont de la précédente vis-à-vis de la direction de la traduction, l'expression de ce fragment étant sous le contrôle d'un promoteur exogène également incorporé de façon stable dans lesdites cellules ou dans le génome de celles-ci.

Dans ce qui suit, il sera quelquefois fait référence à l' "expression fonctionnelle" d'une séquence codant pour GH. Une telle désignation vise à exprimer les idées que la capacité d'expression de la séquence GH

5

au sein de la lignée considérée est essentiellement semblable à celle qui serait réalisée par l'incorporation effective de cette séquence dans le génome des cellules de ces lignées.sous le contrôle d'un promoteur exogène et que cette capacité d'expression.est transmissible aux générations suivantes.

Il est aussi entendu dans ce qui suit que l'on désigne par "protéine ayant les propriétés d'une hormone de croissance" toute protéine ou tout polypeptide capable de manifester son activité dans des tests de reconnaissance spécifique d'une hormone de croissance produite par l'hypophyse du mammifère concerné, notamment dans un test de prolifération dans les conditions décrites par Gout, P.W. et al., 1980, Cancer Research, volume 40, page 2433, et Taraka T. et al., 1980, J. Clin. and Endocrinol. Metab., vol. 51, page 1058, le cas échéant aussi dans un test de croissance du cartilage in vivo chez le rat dans les conditions décrites par Baetzner, P. et al., 1972, Acta Endocrinol., volume 70, pages 231-238, le cas échéant encore dans le test de fixation au récepteur de foie de rat décrit par Kyle, C.N. et al., 1982, Proc. of the Society for Experimental Biology and Medicine, n° 169, pages 368-375.

De même, on désigne par "peptide signal" tout peptide pouvant intervenir au niveau de la capacité de la GH synthétisée dans la cellule transformée de traverser les membranes de cette dernière et d'être excrétée dans le milieu de culture.

On désignera par l'abréviation hGH l'hormone de croissance humaine, ainsi que toute protéine en possédant les propriétés essentielles.

Des séquences préférées codant pour hGH et le peptide signal correspondant sont dérivés d'un cADN codant pour la préhormone de croissance humaine de 217 aminoacides mentionnée plus haut et dont le "peptide signal" consiste en l'extension déjà mentionnée de 26

6

acides aminés, qui est séparée de l'hormone active à l'occasion de son excrétion par les cellules de l'hypophyse. Ces cADN sont susceptibles d'être obtenus de façon en soi connue par transcription reverse de l'ARN messager correspondant et par bicaténarisation de l'ADN obtenu en présence des nucléotides et d'une polymérase appropriée.

On peut également envisager le recours à la séquence d'ADN chromosomique, à condition que celle-ci soit d'abord isolée, notamment par hybridation avec un cADN, excisée de l'ADN chromosomique et enfin incorporée dans un fragment d'ADN, dans les conditions qui seront indiquées plus loin.

Le promoteur exogène mis en oeuvre est distinct ou étranger vis-à-vis du promoteur "endogène" normalement associé au gène codant pour GH dans son hôte naturel, et dont est dérivée la séquence de nucléotides, par exemple le cADN, ultérieurement rendu fonctionnel dans la lignée stable de l'invention. Il est avantageusement choisi en fonction de l'espèce et du tissu dont les cellules réceptrices sont originaires. Lorsque ces cellules sont originaires du singe, il est avantageux d'avoir recours à un promoteur issu du virus SV40, dont est connue la capacité de permettre l'initiation efficace de la transcription de gènes adjacents dans des cellules de singe. Avantageusement, ce promoteur correspond au promoteur "précoce" du virus SV40, lequel contrôle normalement l'expression de l'antigène "petit T" ("small T antigen) et également de l'antigène "grand T" ("large T antigen").

L'invention ne se limite cependant pas à l'utilisation de ce promoteur particulier, bien que celui-ci ait donné des résultats particulièrement favorables eu égard à la production de hGH par les cellules transformées et à son excrétion dans le milieu de culture. On peut également avoir recours par exemple au promoteur

0108667

7

tardif de SV40 (qui contrôle l'expression des protéines VP1, VP2 et VP3). On peut se reporter à la carte de restriction du virus SV40 (J. Tooze, Ed. DNA Tumor Viruses, Cold Spring Harbor Laboratory, Cold Spring Harbor, N.Y., 1980, chaps. 2-5), pour apprécier les positions relatives de ces promoteurs et des gènes codant pour les différents antigènes qui leur sont associés.

Il va de soi que l'on peut substituer aux promoteurs de SV40 tout autre type de promoteur connu comme possédant ou dont pourrait être découverte la capacité de promouvoir la transcription dans les cellules réceptrices de séquences adjacentes ou placées sous son contrôle, avec pour résultats - lorsque ces séquences adjacentes codent pour GH, notamment hGH, et, de préférence aussi, pour un peptide signal qui lui est associé - l'incorporation de ces séquences avec ce promoteur dans le génome des cellules réceptrices et/ou la capacité conférée aux cellules réceptrices ainsi transformées de synthétiser et d'excréter des quantités substantielles de GH, la capacité ainsi acquise étant ensuite transmissible aux générations successives issues de ces cellules.

Lesdites lignées transformées seront dites "stables" lorsque le caractère acquis par les lignées cellulaires selon l'invention de synthétiser GH se transmet d'une génération de cellules à l'autre, sur au moins 10 générations.

De préférence encore le susdit fragment contient également une ou plusieurs des séquences supplémentaires distinctes suivantes :

- séquence (a) contenant au moins une partie susceptible d'être transcrite, sous le contrôle du susdit promoteur exogène, en une partie correspondante d'ARN messager,

- segment (b) contenant un ou plusieurs sites de polyadénylation et de préférence aussi de la terminaison de la transcription de la susdite partie de séquence (a),

8

- séquence (c) contenant au moins un intron, avec les séquences nécessaires à son excision à l'occasion de cette transcription.

La séquence (a) et/ou la séquence (b) sont situées en aval à la fois des susdites première et, le cas échéant, seconde séquences dans le sens de la transcription et, le cas échéant également, de l'intron.

Les positions relatives de l'intron lui-même (séquence (b)) vis-à-vis des susdites première et seconde séquences ne présentent pas un caractère essentiel. L'intron peut être situé en amont ou en aval desdites première et seconde séquences, ou même être inséré dans ces séquences.

Les différentes séquences supplémentaires (a), (b) et/ou (c) sont de la même origine que le promoteur exogène susdit ou d'une origine différente. Elles peuvent en particulier être spécifiques au cADN ou au fragment d'ADN chromosomique codant pour GH. Elles peuvent encore être de différentes origines ; la seule condition, dans le cas de séquences supplémentaires d'origine différente, étant que l'ensemble des séquences ainsi formé ne contienne pas de partie codante pour un signal d'arrêt de la transcription en amont du susdit site de polyadénylation.

Il est en outre préférable, surtout lorsque la ou les séquences supplémentaires sont elles-mêmes originaires de SV40, ou contiennent une ou des parties originaires de SV40, que les parties incluses directement codantes appartenant par exemple au gène "petit T" ou au gène "grand T" (lorsque le promoteur mis en jeu est le promoteur précoce de SV40) soient assez réduites ou interrompues pour que des antigènes complets, à caractère tumorigène, ne soient pas synthétisés par les cellules qui les ont incorporés.

De préférence encore, les séquences supplémentaires sont issues d'ADN susceptibles d'être transcrits et de donner naissance à des protéines ou peptides incapables de posséder un caractère tumorigène. Il est à cet égard avantageux d'avoir recours à une ou des séquences supplémentaires originaires d'un gène d'alpha-globine, par exemple de souris.

Il va de soi que la séquence (b) peut encore, le cas échéant, être prolongée en aval du site de polyadénylation par une séquence supplémentaire correspondant à celle qui, dans le sens de la transcription, suit aussi le site de polyadénylation dans l'ADN dont elle est issue et contient, de préférence, le site de terminaison de la transcription.

De préférence le fragment d'ADN intégré de façon stable dans les lignées selon l'invention contient l'ensemble des séquences (a), (b) et (c), telles qu'elles ont été définies ci-dessus. L'ensemble de ces séquences, lorsqu'elles sont toutes réunies, sera encore désigné sous l'expression "troisième séquence" pour la commodité du langage.

Il est à remarquer que les lignées cellulaires selon l'invention sont capables de synthétiser GH en des quantités importantes rarement atteintes par des cellules de mammifères transformées par les techniques du génie génétique. Sont d'un usage particulièrement avantageux les fragments d'ADN mettant en jeu, à la fois un promoteur précoce de SV40 et, sous le contrôle de ce promoteur, une troisième séquence originaire pour l'essentiel d'une partie au moins du gène codant pour l'alpha-globine de souris, qui contient un intron et un site de polyadénylation, suivi de préférence d'un site de terminaison de la transcription. Les cellules obtenues se sont révélées avoir une capacité de production et d'excrétion de protéines hGH particulièrement importante.

L'invention concerne encore plus particulièrement les lignées de ce type qui possèdent également, intégré dans leur génome, un marqueur de sélection. Celui-ci est plus particulièrement du type des "marqueurs dominants", qui sont aptes à conférer aux cellules transformées la capacité de produire une nouvelle activité enzymatique. Un marqueur préféré de ce type contient une séquence d'ADN codant pour la xanthine-guanine phosphoribosyl-transférase. Le marqueur dominant est de préférence lui-même sous le contrôle direct d'un promoteur distinct du précédent. Ce promoteur distinct peut être identique au premier promoteur mentionné ci-dessus. Il peut également être de nature différente. Dans ce dernier cas, il peut provenir d'un ADN naturel distinct de celui dont avait été extrait le premier promoteur mentionné. Le cas échéant encore, il peut être extrait du même acide nucléique que le premier promoteur. Par exemple, le fragment codant pour la protéine hGH est sous le contrôle du promoteur précoce de SV40 et le marqueur sous le contrôle du promoteur tardif également de SV40.

Le marqueur et le promoteur dont il dépend sont avantageusement associés avec une ou plusieurs séquences distinctes (a), (b) et (c) ou une "troisième séquence" distincte répondant aux définitions qui en ont été données dans ce qui précède, en relation avec les séquences correspondantes éventuellement associées au fragment d'ADN contenant les "première" et "seconde" séquences. Par exemple, le marqueur est également placé sous le contrôle d'un promoteur précoce de SV40 et la séquence transcriptible contient également une séquence (y compris l'intron et les sites de polyadénylation) dont la traduction conduit à la formation d'une partie réduite de l'antigène "petit T".

L'incorporation stable dans le génome des cellules ou l'obtention d'une expression fonctionnelle stable d'un marqueur sous le contrôle d'un promoteur une propriété nouvelle (capacité notamment de synthétiser une nouvelle enzyme) aux cellules selon l'invention. Cette nouvelle propriété permet en particulier leur repérage ou leur sélection. Les cellules isolées grâce à ce marqueur de sélection sont hautement productrices d'hormones de croissance. C'est ce que l'on obtient avec un marqueur constitué par une séquence d'ADN codant pour la xanthine-guanine-phosphoribosyl-transférase, sous le contrôle également d'un promoteur précoce de SV40, et ce plus particulièrement lorsque les cellules sont originaires du singe.

Les lignées selon l'invention sont avantageusement originaires de cellules non tumorigènes. Une lignée préférée est formée par des cellules de rein de singe, par exemple des cellules du type vero ATCC : CCL 81, ou encore par des cellules humaines pour autant que celles-ci puissent être maintenues en culture. On peut aussi, pour la sélection de cellules non tumorigènes de mammifères supérieurs, transformables dans les conditions selon l'invention à l'aide du vecteur décrit ci-après, se reporter utilement aux éditions récentes des catalogues de souches édités par l'American Type Culture Collection (ATCC).

D'une façon générale, on pourra avoir recours à toutes lignées cellulaires non oncogènes, la vérification de ce caractère non oncogène pouvant être effectuée en ayant recours aux procédures recommandées par l'Organisation Mondiale de la Santé (par exemple le test effectué par injection des cellules desdites lignées dans des souris NUDE). La transformation de ces cellules dans les conditions définies par l'invention ne les rendent pas oncogènes, comme on peut le vérifier par la mise en oeuvre des mêmes tests.

Il va de soi que les fragments d'ADN insérés dans les génomes des cellules des lignées selon l'invention peuvent encore présenter les caractéristiques

0108667

12

particulières qui seront encore décrites ci-après, en rapport avec les différentes formes de réalisation de vecteurs applicables à la transformation de cellules d'eucaryotes supérieurs, notamment de mammifères initialement non aptes à excréter, voire même simplement synthétiser une hormone de croissance, en vue de leur conférer ces dernières capacités.

Le vecteur selon l'invention, de préférence un plasmide, est caractérisé en ce qu'il contient en combinaison, d'une part, un promoteur choisi parmi ceux qui sont aptes à s'incorporer de façon stable dans le génome de cellules d'eucaryotes supérieurs, notamment de mammifères, ou de s'y exprimer fonctionnellement de façon stable et, d'autre part, un fragment d'ADN sous le contrôle dudit promoteur, ce fragment contenant une première séquence, de préférence un cADN codant pour une protéine GH, et, de préférence aussi, une seconde séquence codant pour un peptide signal immédiatement en amont de la séquence précédente vis-à-vis de la direction de transcription et, d'autre part encore, l'une au moins des séquences suivantes :

- séquence (a) contenant au moins une partie susceptible d'être transcrite, sous le contrôle du susdit promoteur exogène, en une partie correspondante d'ARN messager,
- segment (b) contenant un ou plusieurs sites de polyadénylation et de préférence aussi de la terminaison de la transcription de la susdite partie de séquence (a),
- séquence (c) contenant au moins un intron, avec les séquences nécessaires à son excision à l'occasion de cette transcription.

Un vecteur préféré de l'invention est celui dans lequel la première séquence code pour hGH. De préférence encore cette première séquence est précédée (vis-à-vis du sens de la transcription) d'une seconde séquence codant pour le peptide précurseur, normalement associé à

13

hGH, lorsque celle-ci est synthétisée dans son hôte naturel sous le contrôle de son promoteur endogène.

Des vecteurs particulièrement préférés, qui codent pour une protéine GH d'origine non humaine, par exemple une GH bovine ou de primate, sont ceux qui comprennent en outre une "troisième séquence" regroupant les séquences (a), (b) et (c) sus-définies.

La troisième séquence de ce vecteur peut donner lieu aux mêmes variantes de constitution, tant en ce qui concerne les natures et les origines de ses différentes régions et de leurs dispositions respectives vis-à-vis desdites première et seconde séquences, que celles qui ont été envisagées en rapport avec les fragments d'ADN incorporés dans le génome des susdites cellules transformées selon l'invention.

Des résultats particulièrement favorables sont obtenus lorsque l'on a recours à une "troisième séquence" originaire du gène de l'alpha-globine de souris. Une "troisième séquence" favorable, qui contient un intron et un site de fixation de poly(A) issu du gène de l'alpha-globine de souris, correspond à celle qui a été décrite par Nishioka, Y. et Leder, P. (1979, Cell 18, 875-882).

De préférence, la partie proximale de la seconde séquence codant pour le peptide signal est très proche, voire directement liée au promoteur, la troisième séquence pouvant alors être placée en son entier en aval de ladite première séquence codant pour GH.

Un vecteur préféré selon l'invention contient un "gène composite" mettant en jeu un cADN cloné comportant toute la séquence codante pour le précurseur de l'hormone de croissance humaine, placée sous le contrôle du promoteur précoce du virus de singe SV40 et suivi d'un fragment d'ADN issu du gène de l'alpha-globine de souris comportant un intron et un site de fixation de poly(A), notamment du type décrit ci-dessus.

14

Les différents vecteurs qui ont été décrits et qui contiennent le gène codant pour GH peuvent être utilisés pour transformer des cellules appropriées, plus particulièrement des cellules de mammifères non tumorigènes. La transformation peut être effectuée dans des conditions en soi connues, par exemple en ayant recours à la technique qui sera rappelée dans les exemples. Les cellules transformées peuvent être repérées ou sélectionnées, par exemple si l'on a eu soin de réaliser cette transformation en présence d'un marqueur de sélection, notamment d'un plasmide apte à transformer les mêmes cellules et contenant une séquence apte à elle-même conférer auxdites cellules la capacité de synthétiser une nouvelle enzyme, par exemple la xanthine-guanine-phosphoribosyl-transférase.

Cependant un vecteur préféré de l'invention est caractérisé par le fait qu'un marqueur de sélection est également intégré de façon covalente dans son ADN. Ce marqueur est avantageusement constitué par un gène codant pour l'enzyme xanthine-guanine-phosphoribosyl-transférase. De préférence encore, ce marqueur de sélection se trouve sous la dépendance d'un promoteur également incorporé de façon stable dans ce vecteur, mais distinct du promoteur associé au fragment contenant le gène codant pour GH. Ce promoteur distinct peut présenter l'une quelconque des différentes caractéristiques qui ont été envisagées à l'occasion de la description des lignées préférées de cellules selon l'invention. Il est de même préférable que ce marqueur de sélection soit incorporé à une "troisième séquence" présentant les mêmes caractéristiques que celle qui est associée avec le gène codant pour GH. Cette dernière séquence correspond avantageusement à celle qui se trouve dans l'acide nucléique initial naturel dont le promoteur choisi est issu. Cette troisième séquence, associée au marqueur peut donner lieu aux mêmes va-

15

riantes de constitution, en ce qui concerne les natures et les origines de ses différentes régions et leurs dispositions relatives vis-à-vis du marqueur, que celles qui ont été envisagées en rapport avec les susdites première et seconde séquences.

Un vecteur préféré selon l'invention contient à la fois le "gène composite" défini plus haut et un fragment distinct comportant un promoteur précoce de SV40, un gène ou cADN codant pour la xanthine-guanine-phosphoribosyl-transférase sous le contrôle de ce promoteur et associé avec une "troisième séquence" contenant une partie de l'ADN originaire de SV40 codant pour une partie de l'antigène "petit T" et le site de polyadénylation correspondant.

L'incorporation d'une séquence codant pour hGH dans ces systèmes de vecteurs tels que définis cidessus, a pour effet d'en optimiser l'apparente efficacité, si l'on en juge par la production et l'excrétion de quantités particulièrement importantes de hGH par les cellules de mammifères supérieurs transformées par le cADN codant pour GH, préalablement introduit dans ces cellules par l'intermédiaire de ces systèmes de vecteurs. La stabilité des transformations que permet le vecteur selon l'invention peut s'apprécier par la conservation du caractère ainsi acquis par les cellules filles, obtenues au cours des générations successives issues des cellules transformées (au moins 10 générations).

Des caractéristiques supplémentaires de l'invention apparaîtront au cours de la description qui suit d'exemples de vecteur particulièrement préférés de l'invention, des conditions dans lesquelles ces vecteurs peuvent être mis en oeuvre pour transformer des cellules de mammifères et des résultats auxquels ladite transformation peut donner lieu et les rendre aptes à synthétiser des GH humaines ou animales, notamment bovines.

16

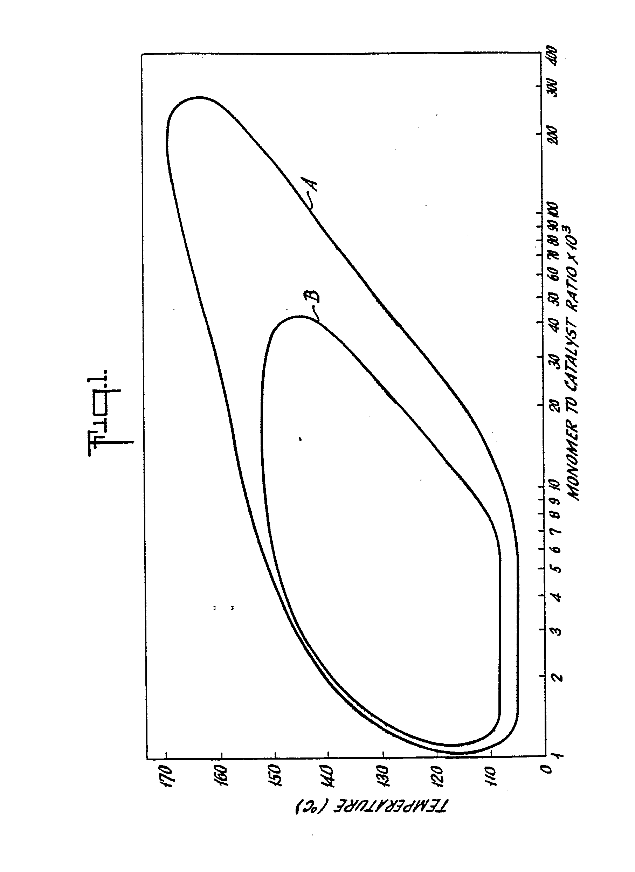

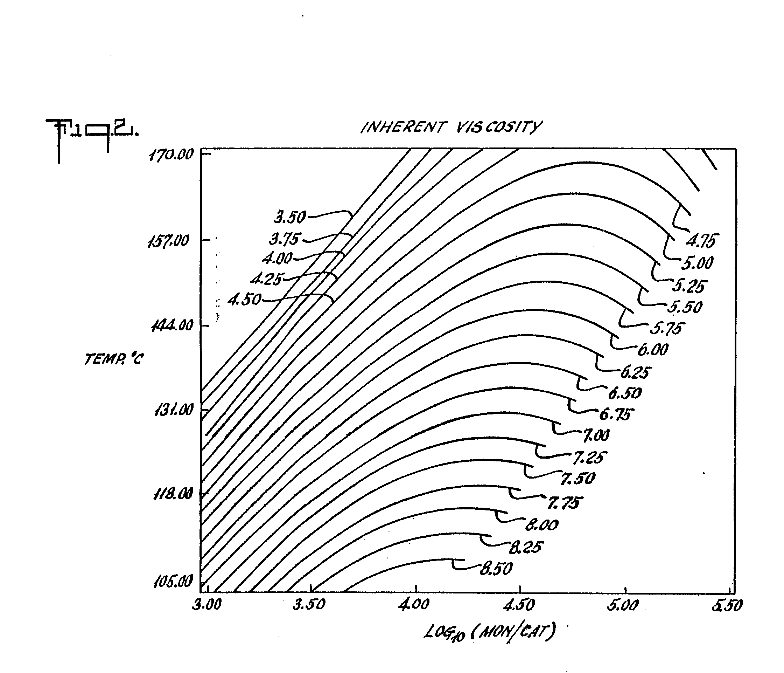

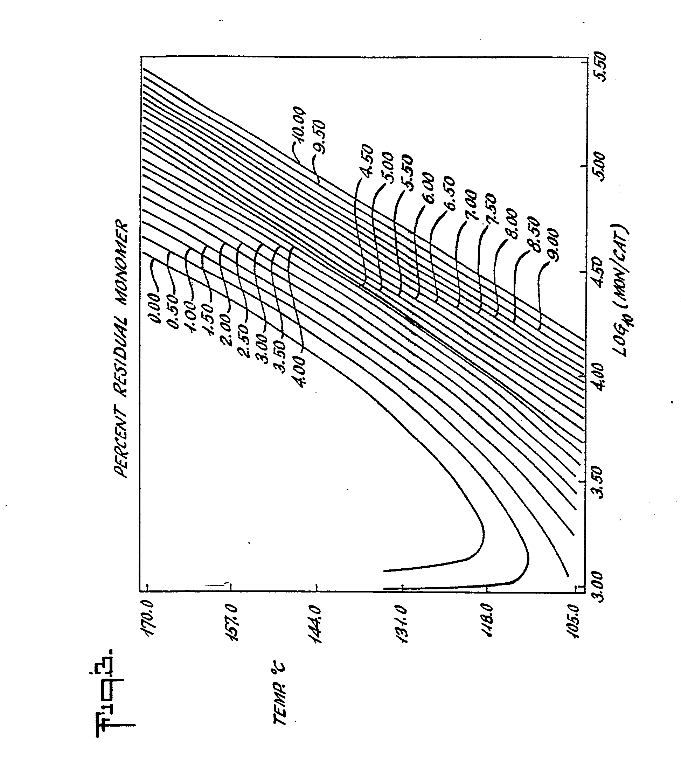

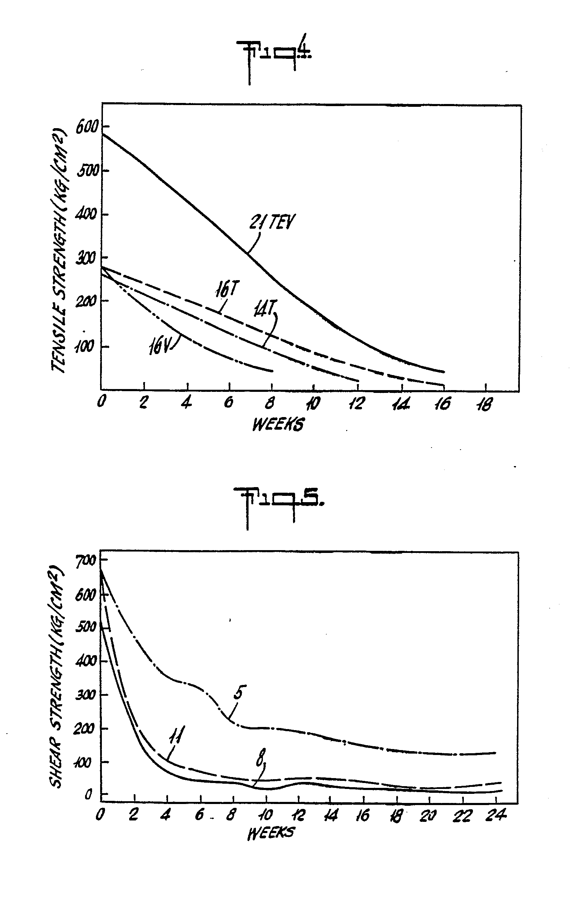

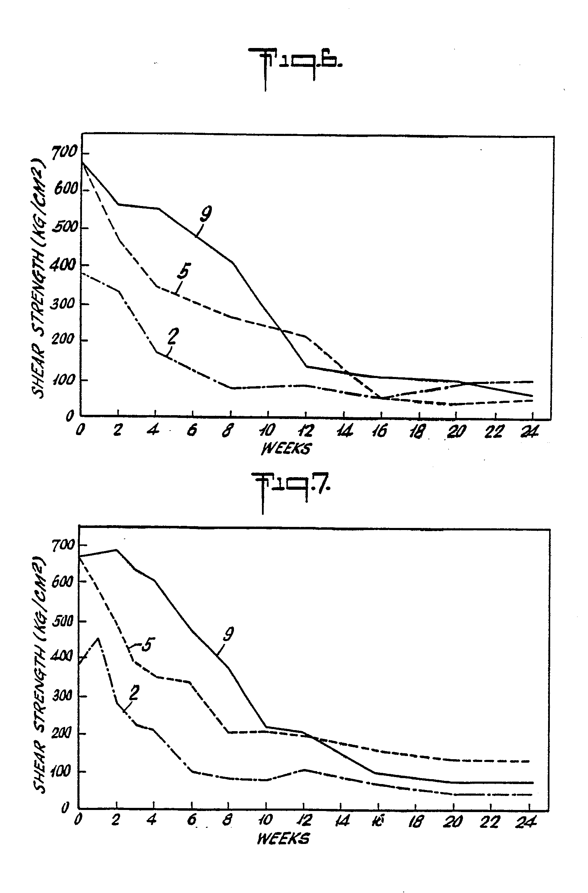

La figure unique jointe illustre de façon schématique les étapes successives de la construction de certains de ces vecteurs.

**Construction de vecteurs modifiés par un insérat codant pour une hGH et production de lignées cellulaires capables de synthétiser et d'excréter cette hGH.**

Le plasmide de départ est constitué par le plasmide pSV498, déposé à la Collection Nationale de Culture de Micro-Organismes de l'Institut Pasteur de Paris (C.N.C.M.) sous le n° I-206. Il a été obtenu par recombinaison, d'une part, du grand fragment isolé sur un gel de polyacrylamide de·3,5 %, après digestion de pSV2gpt, décrit par Mulligan et Berg (1981), Proc. Natl. Acad. Sci. USA 78. 2072-2076, par EcoRI et BamHI et, d'autre part, du fragment de restriction EcoRI-BamHI de 375 paires de bases (p.b.) issu de pBR 322.

Les principaux éléments de structure du plasmide pSV2gpt sont représentés dans la figure unique. Le fragment de restriction de 375 p.b. issu de pBR 322 est représenté en (a). pSV498 diffère donc de pSV2gpt par la substitution du fragment (a) au fragment représenté désigné schématiquement par (b) dans le schéma de pSV2gpt. Ces deux plasmides comprennent en outre en (c) le ·promoteur précoce de SV40, en (d) un gène extrait de E. coli et codant pour la xanthine-guanine-phosphoribosyl-transférase (gène Ecogpt), suivi d'une séquence schématiquement désignée en (e) originaire de SV40 et comprenant une séquence susceptible d'être transcrite en une partie de l'ARN messager correspondant à l'antigène "petit T", un intron et un site de polyadénylation.

pSV498 est coupé partiellement par HindIII (20 microgrammes d'ADN sont incubés avec 5 unités de HindIII pendant 20 minutes à 37°C dans un tampon de 10 mM Tris-HCl, pH 7,6 7 mM Mg Cl2, 60 mM NaCl et 100 microgrammes/

17

millilitre de gélatine).

Après arrêt de la réaction par addition d'EDTA jusqu'à 20 mM final, l'ADN est extrait par un mélange de chloroforme et d'alcool isoamylique, puis précipité à l'éthanol. Il est ensuite incubé en présence de BamHI. Les fragments d'ADN sont séparés par électrophorèse sur un gel de polyacrylamide de 3,5 %. Le fragment de 2779 p.b. (repéré par (f) dans le dessin) est électro-élué. Ce fragment est lié à la séquence du cADN de l'hormone de croissance humaine (Roskam et Rougeon, op. cit.) représenté en (g) contenant un linker (petit fragment de jonction en ADN synthétique) BamHI sur le site SmaI et un linker HindIII sur le site AluI qui précède la séquence codante pour la préhormone. Le plasmide pSV499 est alors obtenu.

pSV499 est coupé par BamHI et lié à un fragment (h) de 593 p.b. contenant la partie distale du gène de l'alpha-globine de souris (Nishioka et Leder, op. cit.) entre les sites MboI situés aux nucléotides 808 et 1401 par rapport au début du mARN (extrait du plasmide p78C16 déposé à la C.N.C.M. sous le n° I-207 et schématiquement représenté en (e). L'orientation de cette insertion est vérifiée après coupure par SacII et ClaI. Un recombinant qui a l'insertion dans le bon sens est nommé pSV500 ; pSV500 est incubé avec ClaI et traité par la polymérase I (fragment de Klenov) pour bicaténariser les extrémités obtenues sous forme de brins simples. Après extraction avec un mélange chloroforme-alcool isoamylique et précipitation à l'éthanol, le plasmide est digéré par PvuI. Un fragment d'environ 3550 p.b. est isolé et lié à un fragment d'environ 3100 p.b. obtenu par incubation de pSV498 avec PvuI et PvuII. Le plasmide obtenu est appelé pSV507.

<u>Expression transitoire de pSV500 et pSV507 dans des cellules vero.</u>

18

Des cultures semi-confluentes de cellules vero (ATCC : CCL81) dans des boites de 60 mm de diamètre sont transfectées avec 10 microgrammes de l'ADN plasmidique par la méthode de DEAE dextran modifiée par Sompayrac L.M. et Danna K.J. (1981) Proc. Natl. Acad. Sci. USA 78, 7575). Après transfection, les cellules sont cultivées dans 2,5 ml de milieu 199 (Morgan, J.F. et al. (1950) Proc. Soc. Explt. Biol. Med. 71, 1) contenant 2 % de sérum de veau foetal pendant 5 jours. La concentration d'hGH excrétée est déterminée par un dosage RIA (kit Institut Pasteur Production). Les quantités sont calculées par pmol d'ADN ajouté (10 microgrammes d'ADN correspondent à 3,6 pmol d'ADN de pSV500 et 2,3 pmol d'ADN de pSV507).

| | |
|---|---|
| pSV500 | 4,3 ng hGH/pmol ADN |
| pSV507 | 10,0 ng hGH/pmol ADN |
| pBR322 | 0 ng hGH/pmol ADN |

Les quantités de hGH produites dans le milieu de culture doivent être considérées comme d'autant plus considérables que seule une partie des cellules ont été transfectées par le vecteur selon l'invention.

Sélection de lignées de cellules vero par le plasmide pSV507.

Environ $1,5 \times 10^5$ cellules vero par boite de 6 cm de diamètre sont transfectées par 7 microgrammes de plasmide pSV507, linéarisé par l'enzyme de restriction PvuI. La méthode utilisée est celle de la précipitation au calcium (Graham et van der Eb (1973) J. Virol. 52, 455-456). Après transfection, les cellules sont cultivées dans du milieu 199 avec 5 % de sérum de veau nouveau-né, pendant 3 jours. Les cellules sont trypsi-nisées et environ $1 \times 10^4$ cellules sont étalées par boite de 10 cm de diamètre. La culture est poursuivie dans un milieu sélectif contenant 10 % de sérum de veau foetal dialysé (milieu sélectif : MEM modifié selon Dulbecco (Dulbecco et Freeman : Virology 8 (1959), 396) contenant 150 microgrammes/ml glutamine, 15 microgrammes/ml hypoxanthine, 2 microgrammes/ml aminoptérine, 250 microgrammes/ml xanthine, 10 microgrammes/ml thymidine et 250 microgrammes/ml ribavérine).

Le milieu est changé deux fois par semaine. Après 3 semaines, des colonies denses sont isolées par trypsination dans des cylindres de verre, et cultivées séparément dans un milieu sélectif contenant 25 microgrammes/ml de ribavérine (permettant la sélection de celles des colonies dont les cellules ont incorporé le plasmide). Les colonies sont ensuite testées pour la production et l'excrétion de l'hormone de croissance humaine par test radio-immunologique.

Plusieurs colonies ont ainsi été obtenues.

Elles peuvent ensuite être cultivées dans un milieu non sélectif (milieu de Eagles modifié par Dulbecco et complété avec du sérum de veau dialysé : 10 % en poids) après adaptation à celui-ci. La lignée qui s'est révélée excréter la plus grande quantité d'hormone hGH a été isolée. Elle a été dénommée VEH1.

Caractérisation de la lignée VEH1.

La lignée VEH1 est étudiée plus particulièrement. La quantité d'hGH synthétisée par cette lignée est importante. Par exemple pendant la phase exponentielle de la croissance en 1 journée, il y a une excrétion de 10 microgrammes d'hGH alors que la quantité de cellules a augmenté de $1,2 \times 10^6$ à $2,7 \times 10^6$ par boite. La lignée VEH1 est stable concernant l'excrétion de l'hGH pendant au moins 11 passages en milieu non sélectif.

Caractérisation de l'hormone excrétée.

Pour déterminer la nature exacte de la protéine produite, les cellules VEH1 sont incubées en présence de la $^{35}S$ méthionine. Le milieu de culture est ensuite so- mis à une immunoprécipitation en utilisant un antisérum de lapin contre l'hGH. L'analyse des précipités sur gel d'acrylamide de 20 % en présence de SDS (Laemli, Nature 227 (1979) 681) révèle une seule bande qui migre exactement à la même position que l'hGH extraite d'hypophyses humaines. Cette bande n'apparaît pas après immunoprécipitation avec un sérum de lapin non immunisé. Pour confirmer qu'il s'agit vraiment de l'hormone de croissance mature et non de la préhormone, les produits d'une traduction in vitro avec de l'ARN codant pour le précurseur de l'hGH sont utilisés comme témoins. Le produit synthétisé in vitro est la préhormone. Les résultats montrent que l'hormone excrétée est l'hormone de croissance et non le précurseur. Les analyses complémentaires ont montré que l'hormone produite est également identique à l'hormone majeure extraite de l'hypophyse sur le plan de la charge, de l'hydrophobicité et de son comportement dans des tests immunologiques quantitatifs.

Le plasmide pSV500 peut encore être modifié pour renforcer son efficacité de production, par référence à la capacité de production de hGH par les cellules transformées. On obtient un tel résultat par remplacement du fragment EcoRI-BamHI (MboI) (provenant de pBR 322) par le fragment EcoRI-BclI (1786-2706) du DNA du virus de singe SV40. Le nouveau plasmide obtenu est nommé pSV511. Du fait de cette modification, le site d'addition de poly(A) pour l'ARN précoce de SV40 (absent dans pSV500) est placé dans une position très proche de la partie distale du gène de l'alpha-globine de souris. Quand des cellules vero sont transfectées avec ce plasmide, pour un test d'expression transitoire, l'excrétion de l'hGH est beaucoup plus importante qu'avec le plasmide de départ (pSV500).

21

Construction d'un plasmide contenant un insérat codant pour une GH bovine (bGH) et production de lignées cellulaires capables de synthétiser et d'excréter cette bGH.

L'hormone de croissance bovine est composée - dans sa forme mature - de 191 acides aminés (Lingappa et al. Proc. Natl. Acad. Sci. USA 74 (1977) 2432-2436). Elle est précédée par un peptide signal de 26 acides aminés (Miller et al. M. Biol. Chem. 255 (1980) 7521-7524). Son enlèvement, pendant l'excrétion, libère l'hormone mature. Le cDNA a été cloné par Miller et al..

On peut aussi faire référence aux séquences de nucléotides codant pour l'hormone de croissance bovine et décrites dans les demandes de brevet européen publiées n° 0067026 et 0075444.

Le clonage du gène de l'hormone de croissance bovine et son expression au sein de cellules de mammifère transformées par ces clones peuvent notamment être réalisés comme suit :

1. Clonage du cDNA dans pBR 322 ;
2. Introduction d'un "linker HindIII" sur le site HphI qui précède le codon d'initiation de la préhormone ;
3. Introduction d'un "linker BamHI" sur le site ScrFI qui suit le codon de terminaison de la préhormone.

On obtient donc la séquence ininterrompue codant pour la bGH, précédée par un site HindIIII et suivie par un site BamHI.

Ce fragment peut être introduit dans pSV498 dans les conditions qui ont été décrites plus haut pour la production de pSV500. Le plasmide obtenu se distingue de pSV500 par le remplacement de hGH par bGH. L'expression transitoire du nouveau plasmide obtenu peut également être réalisée dans les conditions précédemment décrites en rapport avec pSV500.

A partir du nouveau plasmide, on peut construire

22

un plasmide équivalent à pSV507 et l'utiliser pour sélectionner des cellules véro productrices de bGH.

L'invention concerne encore :

- les hGH, telles qu'elles sont produites par les cellules de mammifères transformées selon l'invention,
- les compositions pharmaceutiques contenant ces hGH, en association avec les véhicules pharmaceutiques du type de ceux qui sont utilisés en association avec l'hGH naturelle, ces compositions pharmaceutiques, notamment sous forme injectable ou sous forme prête à la formation extemporanée de solutions injectables, étant utilisables pour les mêmes applications thérapeutiques que l'hormone naturelle d'extraction, notamment pour le traitement du nanisme hypophysaire,
- les anticorps qui peuvent être formés in vivo contre les hGH produites par lesdites cellules de mammifères ; ces anticorps pouvant en particulier, après fixation sur un support insoluble, être utilisés pour la purification d'hGH à partir de mélanges la contenant.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes. A titre d'exemples d'autres promoteurs susceptibles d'être utilisés à la place de ceux issus de SV40, on mentionnera le promoteur tardif d'adénovirus, les promoteurs des gènes codant pour les métallothionéines 1, les promoteurs des rétrovirus, les promoteurs inductibles par la température ou les substances chimiques.

A titre d'exemple d'autres marqueurs, susceptibles d'être utilisés à la place du gène Ecogpt, on peut mentionner ceux codant pour la dihydrofolate réductase de souris, les enzymes de phosphorylation conduisant à l'inactivation de l'antibiotique G 418.

A titre d'exemple d'autre "troisième séquence"

0108667

23

susceptible d'être utilisée à la place de celle issue du gène de l'alpha-globine de souris, on peut mentionner celle issue du gène de la bêta-globine de souris.

A titre d'exemple d'autres "seconde séquence" codant pour un peptide signal, on peut citer ceux codant pour les peptides normalement associés aux albumines et prolactines ou encore à des séquences de nucléotides obtenues par synthèse chimique.

Il est enfin mentionné que toutes les publications auxquelles référence est faite dans cette description sont considérées comme en faisant partie intégrante, dans la mesure où elles concernent les éléments de l'état de la technique servant à l'intelligence complète des bases sur lesquelles a été édifiée l'invention.

0108667

24

## REVENDICATIONS

1 - Lignée stable de cellules de mammifères, notamment de singe, de préférence non tumorigène, dans le génome desquelles est intégré de façon stable, ou dans laquelle s'exprime de façon stable, un fragment d'ADN contenant une première séquence codant pour une protéine ayant les propriétés d'une hormone de croissance et, de préférence aussi, une secone séquence codant pour un peptide signal, immédiatement en amont de la précédente vis-à-vis de la direction de la traduction, l'expression de ce fragment étant sous le contrôle d'un promoteur exogène également présent de façon stable dans lesdites cellules ou dans le génome de celles-ci.

2 - Lignée selon la revendication 1, caractérisée en ce que ledit fragment d'ADN correspond à un cADN ou contient un cADN codant pour une préhormone de croissance humaine.

3 - Lignée selon la revendication 2, caractérisée en ce que la séquence codant pour le peptide signal, code pour celui qui est normalement associé à l'hormone de croissance humaine correspondante.

4 - Lignée selon l'une quelconque des revendications 1 à 3, caractérisée en ce que le promoteur est originaire de SV40, et de préférence correspond au promoteur précoce de SV40.

5 - Lignée selon l'une quelconque des revendications 1 à 4, caractérisée en ce que ledit fragment contient également une ou plusieurs des séquences supplémentaires distinctes suivantes :
- séquence (a) contenant au moins une partie susceptible d'être transcrite, sous le contrôle du susdit promoteur exogène, en une partie correspondante d'ARN messager,
- segment (b) contenant un ou plusieurs sites de polyadénylation et de préférence aussi de la terminaison de la transcription de la susdite partie de séquence (a),

- séquence (c) contenant au moins un intron, avec les séquences nécessaires à son excision à l'occasion de cette transcription.

6 - Lignée selon la revendication 5, caractérisée en ce que le susdit fragment contient une troisième séquence contenant l'ensemble des susdites séquences (a), (b) et (c).

7 - Lignée selon l'une quelconque des revendications 1 à 6, caractérisée en ce que le site de polyadénylation ou l'intron, ou les deux à la fois, sont issus du gène codant pour l'alpha-globine de souris.

8 - Lignée selon l'une quelconque des revendications 1 à 7, caractérisée en ce qu'elle contient également une séquence d'ADN codant pour un marqueur dominant, notamment un gène codant pour la xanthine-guanine phosphoribosyl-transférase, ce marqueur dominant étant de préférence lui-même sous le contrôle direct d'un promoteur distinct du précédent.

9 - Lignée selon la revendication 8, caractérisée en ce que ledit promoteur distinct et le marqueur dominant sont également associés à une ou plusieurs des séquences (a), (b) et (c) telles que définies dans la revendication 5 ou à une "troisième séquence", telle que définie dans la revendication 6.

10 - Lignée selon la revendication 9, caractérisée en ce qu'elle est dérivée de cellules du type vero (ATCC : CCL 81).

11 - Vecteur, tel que plasmide, pour la transformation de cellules d'eucaryotes supérieurs, notamment de mammifère, pour les rendre aptes à produire une protéine ayant les propriétés d'une hormone de croissance, caractérisé en ce qu'il contient en combinaison, d'une part, un promoteur choisi parmi ceux qui sont aptes à s'incorporer de façon stable dans le génome de cellules d'eucaryotes supérieurs, notamment de mammifères, ou de s'y exprimer fonctionnellement de façon stable et, d'autre part, un fragment d'ADN sous le contrôle dudit promoteur, ce fragment contenant une première séquence, de préférence un cADN codant pour une protéine GH, et, de préférence aussi, une seconde séquence codant pour un peptide signal immédiatement en amont de la séquence précédente vis-à-vis de la direction de transcription et, d'autre part encore, l'une au moins des séquences suivantes :

- séquence (a) contenant au moins une partie susceptible d'être transcrite, sous le contrôle du susdit promoteur exogène, en une partie correspondante d'ARN messager,

- segment (b) contenant un ou plusieurs sites de polyadénylation et de préférence aussi de la terminaison de la transcription de la susdite partie de séquence (a),

- séquence (c) contenant au moins un intron, avec les séquences nécessaires à son excision à l'occasion de cette transcription.

12 - Vecteur, selon la revendication 11, caractérisé en ce que la première séquence contient une séquence de nucléotides codant pour hGH.

13 - Vecteur selon la revendication 11 ou la revendication 12, caractérisé en ce qu'il contient une "troisième séquence" contenant l'ensemble des susdites séquences (a), (b) et (c).

14 - Vecteur selon la revendication 13, caractérisé en ce que l'essentiel de la susdite troisième séquence est issue du gène de l'alpha-globine de souris.

15 - Vecteur selon l'une quelconque des revendications 10 à 14, caractérisé en ce que le promoteur est originaire de SV40 et que, de préférence, il consiste en un promoteur précoce de SV40.

16 - Vecteur selon l'une quelconque des reven- dications 11 à 15, caractérisé en ce qu'il contient également un marqueur, de préférence un marqueur dom-

0108667

27

nant apte à induire chez la cellule hôte la capacité à produire une nouvelle activité enzymatique, telle que l'enzyme xanthine-guanine phosphoribosyl-transférase, ce marqueur étant lui-même sous le contrôle d'un promoteur distinct du précédent.

17 - Vecteur selon la revendication 16, caractérisé en ce que ledit promoteur distinct et le marqueur dominant sont associés à une ou plusieurs séquences (a), (b) et (c) telles que définies dans la revendication 13 ou à une "troisième séquence" telle que définie dans la revendicaion 14.

18 - Les protéines ou peptides ayant les propriétés essentielles de l'hormone de croissance, obtenues à partir de cultures de cellules de mammifère , selon l'une quelconque des revendications 1 à 10.

19 - Les compositions pharmaceutiques contenant, en association avec un véhicule pharmaceutique, une protéine conforme à la revendication 18.

20 - Les anticorps contre les protéines selon la revendication 18.

Fig.1.

Fig 2.

INHERENT VISCOSITY

TEMP. °C

LOG₁₀ (MON/CAT)

Fig. 3.

PERCENT RESIDUAL MONOMER

Fig.4

Fig.5.

5/5

Fig.6.

Fig.7.

0108635